# EUROPEAN PATENT SPECIFICATION

(11) **EP 0 497 626 B1**
(45) Date of publication and mention of the grant of the patent: **15.09.1999**
(21) Application number: 92300866.8
(22) Date of filing: 31.01.1992
(51) Int. Cl.: A61K 9/20

(54) **Temperature activated controlled release device**
Temperaturaktivierte Vorrichtung mit geregelter Freigabe
Dispositif à libération contrôlée, activé à la température

(30) Priority: 01.02.1991 US 649381
(43) Date of publication of application: 05.08.1992
(73) Proprietor: E.R. SQUIBB & SONS, INC., Princeton, New Jersey 08543-4000 (US)
(72) Inventor: Kydonieus, Agis, Kendall Park, New Jersey (US); Shah, Kishore R., Bridgewater, New Jersey (US); Decker, Stefanie C., Staten Island, New York (US)
(74) Representative: Nachshen, Neil Jacob

(56) References cited:
- EP-A- 0 317 180
- EP-A- 0 338 732
- EP-A- 0 338 820
- US-A- 4 840 796
- Book no. , , 'TEXTBOOK OF POLYMER SCIENCE', F.W.BILLMEYER JR. ,

## Description

### 1.

This invention relates to a device for the controlled release of biologically active substances and other chemical materials. More particularly, this invention relates to a controlled delivery system for biologically active substances and other chemical materials. As described below, this invention provides a polymeric system for controlled release in which the release of materials is capable of being switched on or off by adjustment of temperature above or below the glass transition temperature of the polymeric material.

### 2. Description of the Prior Art

R.F. Stewart (U.S. Patent 4,830,855 and corresponding EP-A-317 180) has utilized crystalline melting transition temperature (Tm) in a side chain crystallizable polymer to accomplish the temperature activated controlled release of active agents. At temperatures below the Tm of the polymer, the polymer is effectively non-permeable and it acts as a membrane barrier with extremely low diffusional rates for most active agents. In this non-permeable state, the polymer has the ability to protect and contain the desired agent. Above the Tm, the polymer is highly permeable with correspondingly high diffusional rates. The temperature activated release occurs when the polymeric device is heated above its Tm.

T. Okano and coworkers [J. of Contr. Rel. 11, p. 255 (1990)] have described on-off switching polymers for drug permeation and release controlled by temperature change. In this system, a water swollen cross-linked interpenetrating network of poly(N-isopropylacrylamide) and polyurethane is used as the drug matrix. A decrease in temperature of the gel results in higher drug release rates due to "gel squeezing effect".

R. P. Sweet, et al. (U.S. Patent 4,840,796) describe a copolymer drug delivery system having soft and hard segments. The soft segment is polydiorganosiloxane having a low glass transition temperature (Tg) of about -125°C while the hard segment has a high Tg. The copolymer matrix is therefore drug permeable at all practical temperatures, i.e. above -125°C. US Patent 4,840,796 and corresponding EP-A-338732 describe a controlled release device wherein an active agent is loaded into the polymeric device by the copolymer being heated above its Tg (45 to 160°C) and mixed with the active agent. Release of the active agent occuring below the Tg. EP-A-338820 describes a transdermal delivery system comprising a copolymer having a Tg between 50°C and 200°C

### SUMMARY OF THE INVENTION

It has been found that one of the important properties affecting the diffusivity of a solute through a polymeric material is the glass transition temperature (Tg) of the material. Glass transition of a polymer occurs in the temperature region within which the amorphous (noncrystalline) chain segments change from a soft material to a hard brittle solid. A polymer exists in a rubbery state above its Tg. The glass transition region signifies the onset of coordinated motion in a polymer chain. At low temperature, only vibrational motions are possible, and the polymer is hard and glassy. Above the Tg, the chain segments attain sufficient thermal energy to move in a coordinated manner. We have unexpectedly found that when the chain segments move in a coordinated fashion the diffusion through the polymer is magnitudes higher, e.g. a 100 fold increase. We have also unexpectedly found that below the Tg, when there is no coordinated chain segment motion, the diffusion is zero or undetectable. The present invention pertains to devices for the temperature activated release of biologically active substances. More specifically, the active agent release occurs upon raising the temperature of the polymeric device just above its Tg.

The polymeric material used for the device is selected such that its Tg is in the temperature range at which the active agent release is desired. The ambient or normal storage temperatures for the device are below its Tg, under which conditions insignificant release of the active agent occurs due to its very low diffusivity through the polymeric matrix. Upon heating the device above its Tg, greatly increased release of the active agent occurs because of the sudden corresponding increase in diffusivity of the agent through the polymeric matrix. Thus, "on demand" release of the active agent can be affected by means of temperature activation utilizing the phenomenon of glass transition in polymers.

According to one aspect of the invention, there is provided a polymeric device according to claim 1.

According to an alternative embodiment of the invention, there is provided the use of a polymer according to claim 6.

### BRIEF DESCRIPTION OF THE DRAWINGS

Fig. 1 is a cross-sectional view of one embodiment of the device of this invention in which the active agent is dispersed throughout a matrix of rate controlling polymer.
Fig. 2 is a cross-sectional view of another device of this invention in which the active agent is encapsulated by the rate controlling polymer.
Fig. 3 is a cross-sectional view of another device of this invention in which the active agent is dispersed in polymer and the active agent containing polymer matrix is encapsulated by the rate controlling polymer.
Fig. 4 is a graph showing the effect of membrane upon the salicylic acid flux for several rate controlling polymers used in the devices of this invention.

### DESCRIPTION OF PREFERRED EMBODIMENTS

One embodiment of the present invention provides a polymeric device for the temperature activated release of biologically active agents, such that the glass transition temperature (Tg) of the polymer is greater than the normal storage temperatures for the device, the Tg being in the range from 25 to less than 45°C. The release rates of the active agent from the device under storage conditions are very low and negligible. When the device is placed in the desired use environment, sudden release of the active agent occurs at rates which are considerably greater than those under the storage conditions. The effective release rate under the use conditions is governed by solubility and concentration of the active agent in the polymer, and by the device construction.

In the alternative embodiment of the invention where a polymer is used in the preparation of a temperature activated controlled release device, the polymer is selected such that its Tg is below the proposed environmental temperature use.

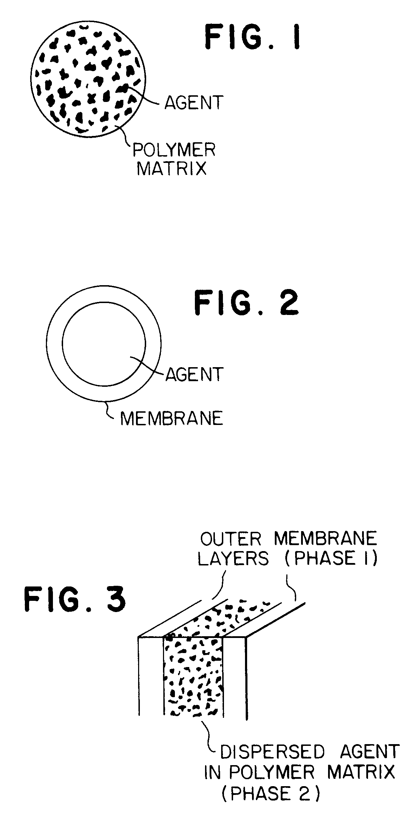

The form of the polymeric devices of this invention can be classified as either monolithic (matrix) (Figure 1),reservoir (Figure 2) or combined monolithic-reservoir (Figure 3). In the monolithic system, the active agent is uniformly dispersed and/or dissolved in the release rate controlling polymer. In the reservoir system, the rate controlling polymer forms the walls or the membrane of a capsule in which the active agent is contained. In the combined monolithic-reservoir system, the active agent is dispersed and/or dissolved in polymer which has its Tg significantly below the use temperature. This active agent containing polymer matrix is encapsulated by the rate controlling polymer. In all of these device embodiments, the rate controlling polymer has its Tg as specified above, its exact value being dependent upon the environmental use temperature for the desired application. The precise shape (slab, sphere, or other) of the device is also governed by requirements of the given application.

The devices of the present invention are suitable for "on-demand" temperature activated controlled release of active agents which include biologically active agents such as therapeutic drugs, antimicrobials, contraceptive agents, pesticides, fungicides, flavors, fragrances, or the like. These devices particularly lend themselves to transdermal drug delivery and for topical application of dermatologically acting agents. In addition, these devices are useful for controlled delivery of medications to wounds. Another potential application can be in subdermally implantable devices which can be activated by externally applied thermal energy. They can also be used internally. Other examples of biologically active agents that may be suited for use in this invention are found in the U.S Patent 4,830,855. The present invention may also be suitably utilized for the controlled release of non-bioactive chemicals or other substances in nonbiologic and/or industrial application. Such a use is controlled release of a catalytic agent in a chemical reaction mixture.

Suitable polymers of use in either embodiment of the present invention may be selected from most of the commercially available polymeric materials such as poly(vinyl acetate), polystyrene, poly(alkyl acrylates), poly(alkyl methacrylates), modified cellulosics, poly(vinyl pyrrolidone), aliphatic polyesters, and vinyl chloride polymers and their copolymers. The Tg of some of the known polymers are shown in Table I but it should be noted that the Tg of polymers for use in accordance with the device of the present invention should be in the range from 25 to less than 45°C. In this respect, for example, the Tg of any and all of these polymeric materials can be altered to the desired range by appropriate plasticization. Additional polymeric materials with their Tg which can be used are described in Polymer Handbook, Third Edition 1989, J. Wiley & Son, J. Brandrup and E. H. Immergut beginning at Page 213. Copolymerization of an alkyl acrylate or an alkyl methacrylate with other members of the acrylate and methacrylate homologous series and/or with other copolymerizable ethylenically unsaturated monomers is a particularly convenient method of preparing materials having the desired Tg. Such copolymers can be prepared by conventional solution, emulsion, or suspension polymerization initiated by free radicals. In addition, copolymers can be formed which are not completely compatible. In this case, two separate Tg's exist and therefore two temperatures for "on-demand" increase in diffusion but both being above the storage or non-use temperature.

The non-rate controlling polymer, containing the active agent for use in the combined monolithic-reservoir devices, is usually a polymer having a low Tg. Examples of such polymers include, but are not limited to, C₂-C₈ alkyl acrylate polymers and their copolymers, poly(vinyl alkyl ethers), polyisobutylene, and polysiloxanes.

In order to ensure the necessary dimensional stability of the device, molecular weights of its polymeric components are usually very high.

The solution or dispersion of the active agent in either the rate controlling or the non-rate controlling polymer, as may be the case, can be prepared by dissolving the agent together with the polymeric component in a suitable solvent and then removing the solvent by evaporation. Such a mixture may also be prepared in a conventional process equipment like a banbury or a sigma-blade mixer under the influence of heat.

The device may be fabricated by employing conventional plastics fabrication methods.

The following Studies are intended to illustrate the permeation of salicyclic acid through three different polymeric membranes, and the controlled release of timolol from poly(n-propyl methacrylate).

### EXAMPLES

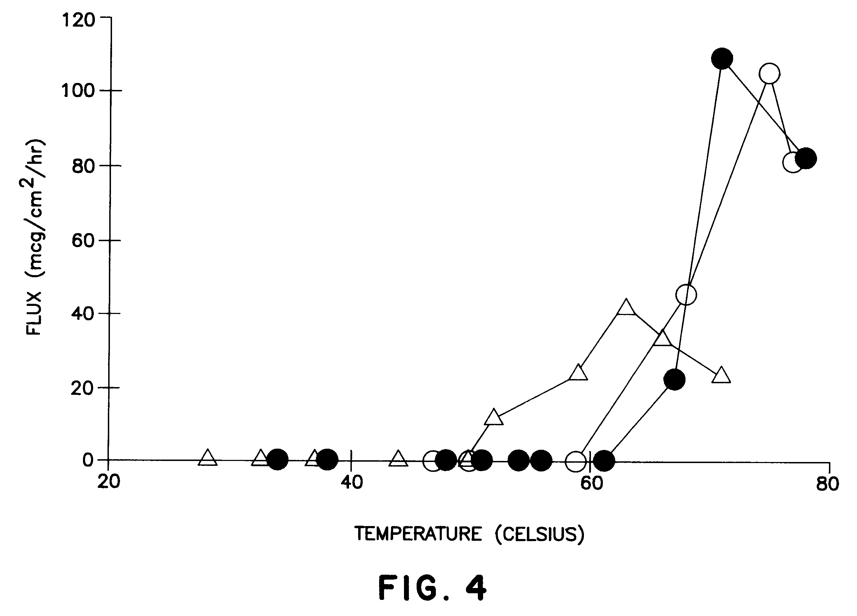

1. Permeation of salicylic acid through three different poLymeric membranes [poly(n-propyl methacrylate), poly(isobutyl methacrylate), and poly(ethyl methacrylate)] at different temperatures was studied, as described below, employing Franz-type diffusion cells.
   A) Materials:
      Poly(n-propyl methacrylate), poly(isobutyl methacrylate) and poly(ethyl methacrylate) were obtained from Scientific Polymer Products, Inc. Salicylic acid from Fisher Scientific was used as the permeant.
   B) Methods:
      DSC Analysis: The Tg of each polymer was measured using a Perkin Elmer DSC-4 Differential Scanning Calorimeter. Samples of polymer were taken directly from the bottle and placed in a sample dish. The scan ranged from 20-120°C and increased at a rate of 10 deg/min. The Tg was determined graphically using the TADS data analysis program.
      Diffusion Experiments: Films for diffusion experiments were prepared by dissolving each polymer in toluene at 30% solids and then casting the solutions on release liner to give 2.5 mil films.
   Diffusion experiments were done using Franz diffusion cells. Three cells per polymer were placed in aluminum blocks in Pierce Reacti-Therm Heating/Stirring Modules which were used to control the temperature. Temperatures were monitored with thermometers set in the aluminum block and insulation material was wrapped around each set of cells.
   Approximately one cc of saturated aqueous salicylic acid solution was placed in the donor compartments of the diffusion cells. The receptor compartment held 7.5 cc of deionized water. The experiments lasted approximately four days during which time the cells were sampled, with complete removal and replacement of receptor solution, several times a day each time raising the block temperature. The samples were analyzed with a Perkin Elmer Lambda 3B UV/VIS Spectrophotometer at wavelength 296 nm.
   The release kinetics were studied for the salicylic acid permeation through each of the membranes at different temperatures. In each case, the system exhibited zero-order kinetics. The permeation rate at each temperature was calculated from the slope of a plot of the cumulative amount permeated versus time. Figure 4 shows the effect of membrane temperature upon the salicylic acid flux for poly(ethyl methacrylate), poly (n-propyl methacrylate), and poly (isobutyl methacrylate), respectively. The maximum in the salicylic acid flux corresponded very closely to the observed Tg of the individual polymeric membranes (Table II) and the onset temperatures for the glass transition corresponded with the permeation starting temperatures.
2. Monolithic controlled release devices were also prepared and tested. Thirty grams of poly (n-propyl methacrylate) and 0.3 grams of timolol base were dissolved in 100 grams of ethyl acetate.
   The solution was cast on release liner and the solvent evaporated. The resulting film was 2.5 mils thick containing 1 percent timolol.
   Diffusion experiments were performed using Franz diffusion cells as described above. the film was placed between the donor and receptor compartments of the diffusion cell with the receptor compartment filled with 7.5 cc of a pH 4.0 phosphate buffer solution. The donor compartment was left empty. At temperatures below 50°C no timolol was detected in the receptor phase. At the temperature of 70°C the release rate of timolol was 4 mg/cm²/hr.

**TABLE I**

| GLASS TRANSITION TEMPERATURES OF SOME COMMON POLYMERIC MATERIALS | |
|---|---|
| POLYMER | Tg. °C |
| Poly(vinyl acetate) | 31 |
| Poly(styrene) | 100 |
| Poly(methyl methacrylate) | 105 |
| Poly(methyl acrylate) | 10 |
| Poly(n-butyl acrylate) | -54 |
| Poly(n-butyl methacrylate) | 20 |
| Poly(vinyl chloride) | 98 |
| Poly(phenyl acrylate) | 57 |
| Poly(cyclohexyl methacrylate) | 83 |
| Poly(3,3-dimethylbutyl methacrylate) | 45 |

**TABLE II**

| EFFECT OF POLYMER GLASS TRANSITION UPON STARTING AND MAX. RATE TEMPERATURES FOR SALICYLIC ACID PERMEATION | | | | |
|---|---|---|---|---|
| Membrane | Temperature, °C | | | |
| | Gloss Transition Onset Temperature | Permeation Start | Tg Obs. (DSC) | Permeation Max |
| Poly(ethyl methacrylate) | 66 | 62 | 76 | 71 |
| Poly(n-propyl methacrylate) | 52 | 50 | 64 | 63 |
| Poly(isobutyl methacrylate) | 62 | 59 | 75 | 73 |

## Claims (Claims for the following Contracting State(s): AT, BE, CH, LI, DE, DK, FR, GB, IT, LU, MC, NL, PT, SE)

1. A polymeric device for the temperature activated release of an active agent comprising a polymer and an active agent, said polymer having a glass transition temperature in the range from 25 to less than 45°C, the device releasing the active agent when placed in an environmental use temperature above the glass transition temperature of the polymer.

2. The polymeric device of claim 1 wherein the device comprises a reservoir containing the active agent to be released and a polymeric membrane surrounding the reservoir.

3. The polymeric device of claim 1 wherein the device comprises a polymer within which the active agent to be released is dissolved or dispersed.

4. The polymeric device of any preceding claim wherein the active agent to be released is a biologically active agent.

5. The polymeric device of any one of claims 1-3 wherein the active agent to be released is a drug, pesticide, fungicide or fragrance.

6. Use of a polymer in the preparation of a temperature activated controlled release device comprising a polymer and an active agent, for the release of the active agent when the device is placed in an environmental use temperature above the glass transition temperature of the polymer.

7. Use according to claim 6 wherein the device comprises a reservoir containing the active agent and a polymeric membrane surrounding the reservoir.

8. Use according to claim 6 wherein the device comprises a polymer within which the active agent is dissolved or dispersed.

9. Use according to any one of claims 6-8 wherein the active agent to be released is a biologically active agent.

10. Use according to any one of claims 6-8 wherein the active agent to be released is a drug, pesticide, fungicide or fragrance.

## Claims (Claims for the following Contracting State(s): ES, GR)

1. A non-pharmaceutical polymeric device for the temperature activated release of an active agent comprising a polymer and an active agent, said polymer having a glass transition temperature in the range from 25 to less than 45°C, the device releasing the active agent when placed in an environmental use temperature above the glass transition temperature of the polymer.

2. The polymeric device of claim 1 wherein the device comprises a reservoir containing the active agent to be released and a polymeric membrane surrounding the reservoir.

3. The polymeric device of claim 1 wherein the device comprises a polymer within which the active agent to be released is dissolved or dispersed.

4. The polymeric device of any preceding claim wherein the active agent to be released is a biologically active agent.

5. The polymeric device of any one of claims 1-3 wherein the active agent to be released is a drug, pesticide, fungicide or fragrance.

6. Use of a polymer in the preparation of a temperature activated controlled release device comprising a polymer and an active agent, for the release of the active agent when the device is placed in an environmental use temperature above the glass transition temperature of the polymer.

7. Use according to claim 6 wherein the device comprises a reservoir containing the active agent and a polymeric membrane surrounding the reservoir wherein the glass transition temperature of said membrane is substantially equal to the temperature desired. for the device to initiate release of the active agent.

8. Use according to claim 6 wherein the device comprises a polymer within which the active agent is dissolved or dispersed wherein the glass transition temperature of said polymer is substantially equal to the temperature desired for the device to initiate release of the active agent.

9. Use according to any one of claims 6-8 wherein the active agent to be released is a drug, pesticide, fungicide or fragrance.

## Patentansprüche (Patentansprüche für folgende(n) Vertragsstaat(en): AT, BE, CH, LI, DE, DK, FR, GB, IT, LU, MC, NL, PT, SE)

1. Polymere Vorrichtung zur temperaturaktivierten Freisetzung eines Wirkstoffes, umfassend ein Polymer und einen Wirkstoff, wobei das Polymer eine Glasübergangstemperatur im Bereich von 25 bis weniger als 45°C aufweist und die Vorrichtung den Wirkstoff freisetzt, wenn sie einer Umgebungsverwendungstemperatur ausgesetzt wird, die über der Glasübergangstemperatur des Polymers liegt.

2. Polymere Vorrichtung gemäß Anspruch 1, wobei die Vorrichtung, ein Reservoir, das den freizusetzenden Wirkstoff enthält, und eine polymere Membran, die das Reservoir umgibt, umfaßt.

3. Polymere Vorrichtung gemäß Anspruch 1, wobei die Vorrichtung ein Polymer umfaßt, in dem der freizusetzende Wirkstoff gelöst oder dispergiert ist.

4. Polymere Vorrichtung nach einem der vorstehenden Ansprüche, wobei der freizusetzende Wirkstoff ein biologischer Wirkstoff ist.

5. Polymere Vorrichtung nach einem der Ansprüche 1 bis 3, wobei der freizusetzende Wirkstoff ein Arzneimittel, Pestizid, Fungizid oder Duftstoff ist.

6. Verwendung eines Polymers für die Herstellung einer temperaturaktivierten Vorrichtung zur geregelten Freisetzung, umfassend ein Polymer und einen Wirkstoff zur Freisetzung des Wirkstoffs wenn die Vorrichtung einer Umgebungsverwendungstemperatur ausgesetzt wird, die über der Glasübergangstemperatur des Polymers liegt.

7. Verwendung gemäß Anspruch 6, wobei die Vorrichtung ein Reservoir, das den Wirkstoff enthält, und eine polymere Membran, die das Reservoir umgibt, umfaßt.

8. Verwendung gemäß Anspruch 6, wobei die Vorrichtung ein Polymer umfaßt, in dem der Wirkstoff gelöst oder dispergiert ist.

9. Verwendung nach einem der Ansprüche 6 bis 8, wobei der freizusetzende Wirkstoff ein biologischer Wirkstoff ist.

10. Verwendung nach einem der Ansprüche 6 bis 8, wobei der freizusetzende Wirkstoff ein Arzneimittel, Pestizid, Fungizid oder Duftstoff ist.

## Patentansprüche (Patentansprüche für folgende(n) Vertragsstaat(en): ES, GR)

1. Nicht pharmazeutische polymere Vorrichtung zur temperaturaktivierten Freisetzung eines Wirkstoffes, umfassend ein Polymer und einen Wirkstoff, wobei das Polymer eine Glasübergangstemperatur im Bereich von 25 bis weniger als 45°C aufweist und die Vorrichtung den Wirkstoff freisetzt, wenn sie einer Umgebungsverwendungstemperatur ausgesetzt wird, die über der Glasübergangstemperatur des Polymers liegt.

2. Polymere Vorrichtung gemäß Anspruch 1, wobei die Vorrichtung, ein Reservoir, das den freizusetzenden Wirkstoff enthält, und eine polymere Membran, die das Reservoir umgibt, umfaßt.

3. Polymere Vorrichtung gemäß Anspruch 1, wobei die Vorrichtung ein Polymer umfaßt, in dem der freizusetzende Wirkstoff gelöst oder dispergiert ist.

4. Polymere Vorrichtung nach einem der vorstehenden Ansprüche, wobei der freizusetzende Wirkstoff ein biologischer Wirkstoff ist.

5. Polymere Vorrichtung nach einem der Ansprüche 1 bis 3, wobei der freizusetzende Wirkstoff ein Arzneimittel, Pestizid, Fungizid oder Duftstoff ist.

6. Verwendung eines Polymers für die Herstellung einer temperaturaktivierten Vorrichtung zur geregelten Freisetzung, umfassend ein Polymer und einen Wirkstoff zur Freisetzung des Wirkstoffs wenn die Vorrichtung einer Umgebungsverwendungstemperatur ausgesetzt wird, die über der Glasübergangstemperatur des Polymers liegt.

7. Verwendung gemäß Anspruch 6, wobei die Vorrichtung ein Reservoir, das den Wirkstoff enthält, und eine polymere Membran, die das Reservoir umgibt, umfaßt, wobei die Glasübergangstemperatur der Membran im wesentlichen gleich ist mit der Temperatur, die gewünscht ist, so daß die Vorrichtung anfängt den Wirkstoff freizusetzen.

8. Verwendung gemäß Anspruch 6, wobei die Vorrichtung ein Polymer umfaßt, in dem der Wirkstoff gelöst oder dispergiert ist, wobei die Glasübergangstemperatur des Polymers im wesentlichen gleich ist mit der Temperatur, die gewünscht ist, so daß die Vorrichtung anfängt den Wirkstoff freizusetzen.

9. Verwendung nach einem der Ansprüche 6 bis 8, wobei der freizusetzende Wirkstoff ein Arzneimittel, Pestizid, Fungizid oder Duftstoff ist.

## Revendications (Revendications pour l'(les) Etat(s) contractant(s) suivant(s): AT, BE, CH, LI, DE, DK, FR, GB, IT, LU, MC, NL, PT, SE)

1. Dispositif polymère permettant la libération activée par la température d'un agent actif, comprenant un polymère et un agent actif, ledit polymère ayant une température de transition vitreuse située dans l'intervalle de 25 à moins de 45°C, ce dispositif libérant l'agent actif quand il est placé dans un milieu où règne une température d'utilisation supérieure à la température de transition vitreuse du polymère.

2. Dispositif polymère selon la revendication 1, dans lequel le dispositif comprend un réservoir contenant l'agent actif à libérer et une membrane polymère entourant ce réservoir.

3. Dispositif polymère selon la revendication 1, dans lequel le dispositif comprend un polymère au sein duquel l'agent actif à libérer est dissous ou dispersé.

4. Dispositif polymère selon l'une quelconque des revendications précédentes, dans lequel l'agent actif à libérer est un agent biologiquement actif.

5. Dispositif polymère selon l'une quelconque des revendications 1 à 3, dans lequel l'agent actif à libérer est un médicament, un pesticide, un fongicide ou un parfum.

6. Utilisation d'un polymère dans la préparation d'un dispositif de libération contrôlée activé par la température comprenant un polymère et un agent actif, permettant la libération de l'agent actif quand le dispositif est placé dans un milieu où règne une température d'utilisation supérieure à la température de transition vitreuse du polymère.

7. Utilisation selon la revendication 6, dans laquelle le dispositif comprend un réservoir contenant l'agent actif et une membrane polymère entourant ce réservoir.

8. Utilisation selon la revendication 6, dans laquelle le dispositif comprend un polymère au sein duquel l'agent actif est dissous ou dispersé.

9. Utilisation selon l'une quelconque des revendications 6 à 8, dans laquelle l'agent actif à libérer est un agent biologiquement actif.

10. Utilisation selon l'une quelconque des revendications 6 à 8, dans laquelle l'agent actif à libérer est un médicament, un pesticide, un fongicide ou un parfum.

## Revendications (Revendications pour l'(les) Etat(s) contractant(s) suivant(s): ES, GR)

1. Dispositif polymère non pharmaceutique permettant la libération activée par la température d'un agent actif, comprenant un polymère et un agent actif, ledit polymère ayant une température de transition vitreuse située dans l'intervalle de 25 à moins de 45°C, ce dispositif libérant l'agent actif quand il est placé dans un milieu où règne une température d'utilisation supérieure à la température de transition vitreuse du polymère.

2. Dispositif polymère selon la revendication 1, dans lequel le dispositif comprend un réservoir contenant l'agent actif à libérer et une membrane polymère entourant ce réservoir.

3. Dispositif polymère selon la revendication 1, dans lequel le dispositif comprend un polymère au sein duquel l'agent actif à libérer est dissous ou dispersé.

4. Dispositif polymère selon l'une quelconque des revendications précédentes, dans lequel l'agent actif à libérer est un agent biologiquement actif.

5. Dispositif polymère selon l'une quelconque des revendications 1 à 3, dans lequel l'agent actif à libérer est un médicament, un pesticide, un fongicide ou un parfum.

6. Utilisation d'un polymère dans la préparation d'un dispositif de libération contrôlée activé par la température comprenant un polymère et un agent actif, permettant la libération de l'agent actif quand le dispositif est placé dans un milieu où règne une température d'utilisation supérieure à la température de transition vitreuse du polymère.

7. Utilisation selon la revendication 6, dans laquelle le dispositif comprend un réservoir contenant l'agent actif et une membrane polymère entourant ce réservoir, dans laquelle la température de transition vitreuse de ladite membrane est sensiblement égale à la température voulue pour que le dispositif déclenche la libération de l'agent actif.

8. Utilisation selon la revendication 6, dans laquelle le dispositif comprend un polymère au sein duquel l'agent actif est dissous ou dispersé, dans laquelle la température de transition vitreuse dudit polymère est sensiblement égale à la température voulue pour que le dispositif déclenche la libération de l'agent actif.

9. Utilisation selon l'une quelconque des revendications 6 à 8, dans laquelle l'agent actif à libérer est un médicament, un pesticide, un fongicide ou un parfum.
